(19) Europäisches Patentamt
European Patent Office
Office européen des brevets

(11) Veröffentlichungsnummer : **0 075 866**
**B1**

(12) # EUROPÄISCHE PATENTSCHRIFT

(45) Veröffentlichungstag der Patentschrift : **19.07.89**

(51) Int. Cl.$^4$ : **C 07 D307/93**, C 07 D311/94, C 11 B 9/00

(21) Anmeldenummer : **82108798.8**

(22) Anmeldetag : **23.09.82**

(54) **Substituierte macrobicyclische Ether, deren Herstellung und Verwendung.**

(30) Priorität : **24.09.81 DE 3137939**

(43) Veröffentlichungstag der Anmeldung : **06.04.83 Patentblatt 83/14**

(45) Bekanntmachung des Hinweises auf die Patenterteilung : **19.07.89 Patentblatt 89/29**

(84) Benannte Vertragsstaaten : **CH DE FR GB LI NL**

(56) Entgegenhaltungen :
**EP–A– 0 004 052**
**DE–A– 2 136 496**

(73) Patentinhaber : **Consortium für elektrochemische Industrie GmbH
Zielstattstrasse 20
D-8000 München 70 (DE)**

(72) Erfinder : **Gebauer, Helmut, Dr.
Schaffhauser Strasse 18/7
D-8000 München 71 (DE)**

**EP 0 075 866 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

In der DE-OS 28 10 107 wird 13-Oxabicyclo [10.3.0] pentadecan, eine Verbindung, die zu den macrobicyclischen Ethern zu zählen ist, beschrieben. Die genannte Verbindung ist in einem 4-Stufen-Prozeß, ausgehend von Cyclododecanon und Bromessigester zugänglich. Sie findet Verwendung als Riechstoff mit warmer Ambranote.

Aufgabe der Erfindung war es, neue Riechstoffe aufzufinden.

Es wurde nun in einer Auswahl von alkyl- und/oder alkenylsubstituierten 13-Oxabicyclopentadecanen und 13-Oxabicyclohexadecanen eine Gruppe von Riechstoffen gefunden, die ein breites Spectrum verschiedener Geruchsnoten abdeckt.

Gegenstand der Erfindung sind demnach Verbindungen der allgemeinen Formel I

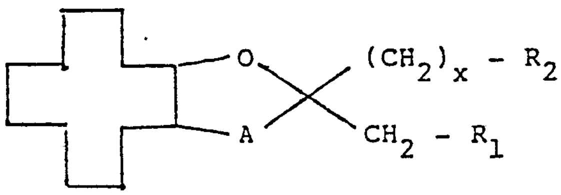

(I)

in der

$R_1$ und $R_2$ Wasserstoff oder ggf. verzweigte Alkylgruppen mit 1 bis 6 Kohlenstoffatomen oder ggf. verzweigte Alkenylgruppen mit 3 bis 6 Kohlenstoffatomen bedeuten,

A für einen zweiwertigen Kohlenwasserstoffrest der allgemeinen Formel II

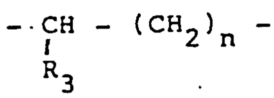

(II)

steht, in der

$R_3$ Wasserstoff oder Methyl bedeutet und

n und x den jeweils gleichen numerischen Wert von 0 oder 1 haben.

Bevorzugt sind die erfindungsgemäßen Verbindungen mit 15 bis 21 Kohlenstoffatomen pro Molekül, besonders bevorzugt die Verbindungen mit 15 bis 17 Kohlenstoffatomen pro Molekül.

Entsprechend bedeuten $R_1$ und $R_2$ bevorzugt Wasserstoff oder Methyl.

Die Erfindung umfaßt sowohl 13-Oxabicyclo [10.3.0] pentadecane der allgemeinen Formel Ia

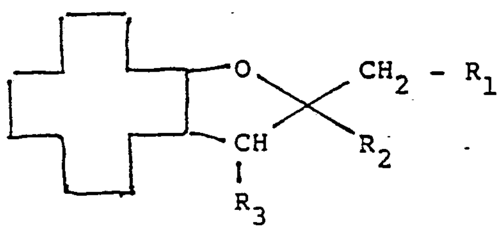

(Ia)

als auch 13-Oxabicyclo [10.4.0] hexadecane der allgemeinen Formel Ib

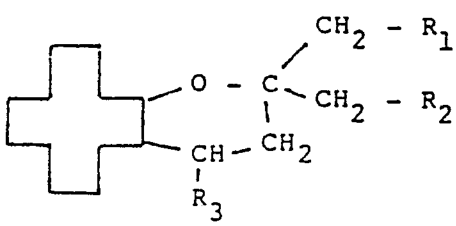

(Ib)

Beispiele für erfindungsgemäße Verbindungen sind :

14-Methyl-13-oxabicyclo [10.3.0] pentadecan
14.14-Dimethyl-13-oxabicyclo [10.3.0] pentadecan
14-Ethyl-13-oxabicyclo [10.3.0] pentadecan

14-Propyl-13-oxabicyclo [10.3.0] pentadecan
14-Isopropyl-14-methyl-13-oxabicyclo [10.3.0] pentadecan
14-Butyl-13-oxabicyclo [10.3.0] pentadecan
14-Pentyl-13-oxabicyclo [10.3.0] pentadecan
14-Isopentyl-13-oxabicyclo [10.3.0] pentadecan
14-Hexyl-13-oxabicyclo [10.3.0] pentadecan
14-Isohexyl-13-oxabicyclo [10.3.0] pentadecan
14.15-Dimethyl-13-oxabicyclo [10.3.0] pentadecan
14-Ethyl-15-methyl-13-oxabicyclo [10.3.0] pentadecan
14-Propyl-15-methyl-13-oxabicyclo [10.3.0] pentadecan
14-Isobutyl-15-methyl-13-oxabicyclo [10.3.0] pentadecan

14-(But-3-en-1-yl)-13-oxabicyclo [10.3.0] pentadecan
14-(But-3-en-1-yl)-15-methyl-13-oxabicyclo [10.3.0] pentadecan
14-(Pent-4-en-1-yl)-13-oxabicyclo [10.3.0] pentadecan
14-Methyl-14-(2-methyl-pent-4-en-1-yl)-13-oxabicyclo [10.3.0] pentadecan
14.15-Dimethyl-14-(2-methyl-pent-4-en-1-yl)-13-oxabicyclo [10.3.0] pentadecan
14.14-Diethyl-13-oxabicyclo [10.3.0] pentadecan
14.14.15-Trimethyl-13-oxabicyclo [10.3.0] pentadecan
14.14-Diethyl-15-methyl-13-oxabicyclo [10.3.0] pentadecan
14.14-Dimethyl-13-oxabicyclo [10.4.0] hexadecan
14.14-Diethyl-13-oxabicyclo [10.4.0] hexadecan
14.14-Dipropyl-13-oxabicyclo [10.4.0] hexadecan
14.14.16-Trimethyl-13-oxabicyclo [10.4.0] hexadecan
14.14-Diethyl-16-methyl-13-oxabicyclo [10.4.0] hexadecan
14.16-Dimethyl-15-ethyl-13-oxabicyclo [10.4.0] hexadecan
14-Ethyl-14-methyl-13-oxabicyclo [10.4.0] hexadecan
14-Propyl-14-methyl-13-oxabicyclo [10.4.0] hexadecan
14-Propenyl-14-methyl-13-oxabicyclo [10.4.0] hexadecan
14-Ethyl-14.16-dimethyl-13-oxabicyclo [10.4.0] hexadecan
14.14-Diethyl-16-methyl-13-oxabicyclo [10.4.0] hexadecan
14-Propenyl-14.16-dimethyl-13-oxabicyclo [10.4.0] hexadecan
14-(4-Methyl-pent-3-en-1-yl)-14-methyl-13-oxabicyclo [10.4.0] hexadecan

Die erfindungsgemäßen Verbindungen sind durch intramolekulare Ringschlußreaktion von Cyclododecanolen zugänglich, die in der 2-Ringposition β-γ-ungesättigte Substituenten aufweisen.

Im Falle solcher substituierten Cyclododecanole, die in der γ-Stellung des obengenannten Substituenten mindestens ein Vinylwasserstoff atom aufweisen, entstehen die entsprechenden 13-Oxabicyclopentadecane, während bei analogen Verbindungen ohne Vinylwasserstoff atom in der γ-Stellung die entsprechenden 13-Oxabicyclohexadecane gewonnen werden.

Ein bevorzugtes Verfahren zur Herstellung der erfindungsgemäßen 13-Oxabicyclo [10.3.0] pentadecane ist dadurch gekennzeichnet, daß Verbindungen der allgemeinen Formel III

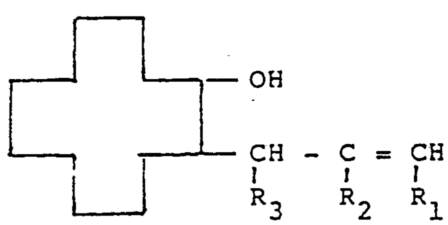

(III)

in der $R_1$, $R_2$ und $R_3$ die oben angegebene Bedeutung haben, bei Temperaturen von 20 bis 150 °C mit einer Säure behandelt werden.

Die Säure wird, bezogen auf die Menge des zur Reaktion zu bringenden Dodecanols in Mengen von 1 bis 20 Mol% eingesetzt.

Beispiele für erfindungsgemäß einzusetzende Säuren sind anorganische Säuren, wie Salzsäure, Schwefelsäure und Phosphorsäure. Insbesondere werden jedoch organische Säuren, wie beispielsweise p-Toluolsulfonsäure, eingesetzt.

Zumeist wird in inerten Lösungsmitteln, wie Benzol, Toluol oder Xylolen, cyclisiert. Es kann jedoch auch ohne Lösungsmittel gearbeitet werden.

Das bevorzugte Herstellungsverfahren für die erfindungsgemäßen 13-Oxabicyclo [10.4.0] hexadecane ist dadurch gekennzeichnet, daß Verbindungen der allgemeinen Formel IIIa

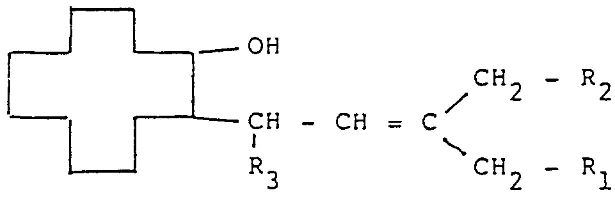

(IIIa)

bei Temperaturen von 20 bis 150 °C mit einer Säure behandelt werden. Im übrigen erfolgt die Herstellung der erfindungsgemäßen Hexadecane analog der oben beschriebenen Herstellung der erfindungsgemäßen Pentadecane.

Die Herstellung der als Ausgangsverbindungen einzusetzenden alkylierten Cyclododecanole erfolgt vorzugsweise in einem 2-Stufen-Prozeß, ausgehend von dem im Handel erhältlichen Cyclododecanon.

In der ersten Reaktionsstufe wird Cyclododecanon in α-Stellung mit einem allylischem Halogenid alkyliert. Ist als Zielprodukt ein erfindungsgemäßes Bicyclopentadecan erwünscht, wird ein allylisches Halogenid der allgemeinen Formel IV

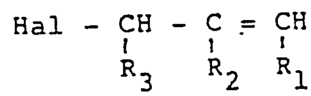

(IV)

eingesetzt. Für die Synthese der erfindungsgemäßen Bicyclohexadecane wird ein allylisches Halogenid der allgemeinen Formel V

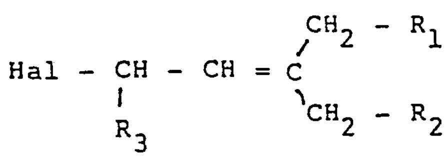

(V)

verwendet.

Hal steht in den letztgenannten allgemeinen Formeln jeweils für ein Chlor-, Brom- oder Jod-, insbesondere für ein Chloratom.

Spezielle Beispiele für allylische Halogenide sind Allylchlorid, Methallylchlorid, Crotylchlorid, Prenylchlorid, Geranylchlorid und 3-Chlor-1-Buten.

Die erfindungsgemäß einzusetzenden allylischen Halogenide sind zumeist im Handel erhältlich oder sind über Aldolkondensationsprodukte oder durch Anlagerung von Halogenwasserstoff an konjugierte C-C-Doppelbindungen zugänglich.

Die α-Alkylierung von Cyclododecanon kann in herkömmlicher Weise in Gegenwart starker organischer Basen mit sperrigen Resten, wie beispielsweise Kalium-tert.-Butylat oder Natrium-tert.-Amylat, durchgeführt werden. Gewöhnlich wird so verfahren, daß ein äquimolares Gemisch von Cyclododecanon und allylischem Halogenid in inertem Lösungsmittel, wie Toluol, vorgelegt wird und äquimolare Mengen einer organischen Base zudosiert werden. Die Reaktionstemperaturen liegen in der Regel bei 60 bis 110 °C.

Überraschenderweise führen auch sogenannte phasentransferkatalysierte Alkylierungen zu guten Ausbeuten. Dabei werden Cyclododecanon und allylisches Halogenid in einem organisch/alkalischen 2-Phasen-System in Gegenwart eines Phasentransferkatalysators umgesetzt.

Das 2-Phasen-System wird gebildet aus einem organischen, mit Wasser nicht mischbaren, inerten Lösungsmittel und einer 5 bis 50 %-igen wäßrigen Lösung oder in fester Form vorliegendem Alkalimetallhydroxid.

Beispiele für inerte Lösungsmittel sind Benzol, Toluol, Xylole, Cyclohexan, Petrolether und Benzine. Es können ebenso Gemische davon eingesetzt werden.

Beispiele für Alkalimetallhydroxide sind NaOH und KOH. Bezogen auf eingesetztes Alkylierungsmittel sind äquimolare Mengen an Lauge erforderlich — reaktionsbeschleunigend wirkt jedoch ein etwa 2-facher Überschuß an Lauge.

Es können Phasentransferkatalysatoren eingesetzt werden, die auch bereits bisher zu derartigen Reaktionen verwendet wurden. Beispiele sind Kronenether, quartäre Ammonium- und Phosphoniumsalze, insbesondere Tetra-butyl-ammonium-bromid. Die Katalysatoren werden in Mengen von 0,5 bis 5 Mol%, bezogen auf allylisches Halogenid, eingesetzt, wobei sich 2 bis 3 Mol% in der Regel als am wirtschaftlichsten erweisen.

Vorteilhafterweise werden das 2-Phasen-System, Cyclododecanon und Phasentransferkatalysator vorgelegt und allylisches Halogenid zugetropft. Bei dieser Arbeitsweise können Zweifachalkylierungen weitgehendst vermieden werden.

Die Reaktionstemperaturen betragen im allgemeinen zwischen 0 und 150 °C, bevorzugt 20 bis 110 °C. Oftmals wird ein optimales Verhältnis von Reaktionszeit und Ausbeute bei Temperaturen zwischen 60 und 80 °C erzielt.

Die Aufarbeitung des Reaktionsgemisches kann durch konventionelle Techniken erfolgen : gewöhnlich trennt man die Phasen und unterwirft, zur Isolierung des gewünschten Produkts, die organische Phase einer fraktionierten Destillation.

Als zweite Reaktionsstufe schließt sich die Reduktion des in α-Stellung alkylierten Cyclododecanons zum entsprechenden Cyclododecanol an. Es werden Reduktionsmittel eingesetzt, die spezifisch die Carbonylfunktion reduzieren, ohne die olefinische Doppelbindung in β-γ-Stellung des Substituenten anzugreifen.

Beispiele für solche Reduktionsmittel sind komplexe Hydride, wie Natriumboranat oder Lithiumalanat.

Alternativ läßt sich die Reduktion auch in an sich bekannter Weise nach Meerwein-Ponndorf-Verley durchführen, wobei mit Isopropanol in Gegenwart von Aluminium-Isopropylat reduziert wird.

Demnach ist ein besonders bevorzugtes Verfahren zur Herstellung der Verbindungen der allgemeinen Formel I

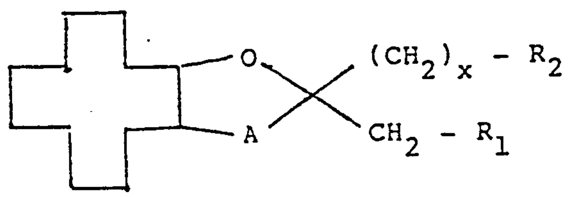

(I)

in der

$R_1$ und $R_2$ Wasserstoff oder ggf. verzweigte Alkylgruppen mit 1 bis 6 Kohlenstoffatomen oder ggf. verzweigte Alkenylreste mit 3 bis 6 Kohlenstoffatomen bedeuten,

A einen zweiwertigen Kohlenwasserstoffrest der allgemeinen Formel II

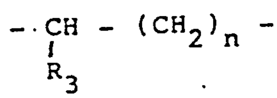

(II)

bedeutet,

$R_3$ Wasserstoff oder Methyl bedeutet und

n und x den jeweils gleichen numerischen Wert von 0 oder 1 haben,

dadurch gekennzeichnet, daß

a1) mit der Maßgabe, daß x und n identisch 0 sind, Cyclododecan on mit einem allylischen Halogenid der allgemeinen Formel IV

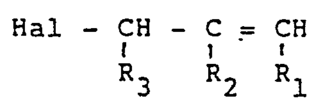

(IV)

in der

Hal für ein Chlor-, Brom- oder Jodatom steht und

$R_1$, $R_2$ und $R_3$ die oben angegebene Bedeutung haben

a2) mit der Maßgabe, daß x und n identisch 1 sind, Cyclododecanon mit einem allylischen Halogenid der allgemeinen Formel V

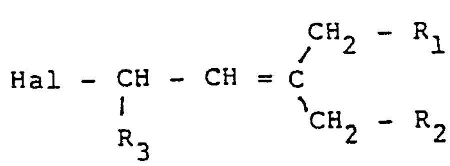

(V)

mit der oben angegebenen Bedeutung für Hal, $R_1$, $R_2$ und $R_3$ in einem organisch/alkalischen 2-Phasen-System in Gegenwart eines Phasentransferkatalysators umgesetzt wird,

b) das Reaktionsprodukt gemäß a1) bzw. a2) mit Isopropanol in Gegenwart von Aluminiumisopropylat zum entsprechenden Alkohol reduziert wird und

c) das Reaktionsprodukt gemäß b) bei Temperaturen von 20 bis 150 °C einer Säurebehandlung unterworfen wird.

Die erfindungsgemäßen Verbindungen finden Verwendung als Riechstoffe. Innerhalb der Gruppe der

erfindungsgemäßen Verbindungen tritt ein breites Spektrum verschiedenartigster Geruchsnuancen auf, vom warmen, vetiverartigen Sandelholzton bis zur holzigen Moschusnote.

Sämtliche erfindungsgemäßen Verbindungen können als Fixateure eingesetzt werden. Sie verfügen ferner über ausgezeichnete Hafteigenschaften.

So können die erfindungsgemäßen Verbindungen einzeln oder im Gemisch oder im Gemisch mit anderen bekannten Riechstoffen beispielsweise als Bestandteile von Parfums oder zur sensorischen Verbesserung einer Vielzahl von Produkten, einschließlich Farbdispersionen, eingesetzt werden.

Die Erfindung wird nun anhand von Beispielen näher erläutert :

## Beispiel 1

Herstellung von 14-Methyl-13-oxabicyclo [10.3.0] pentadecan

a) In einem 500 ml Dreihalskolben mit Rührer, Rückflußkühler und Tropftrichter werden 40 g (1 Mol) Natriumhydroxid-Plätzchen, 182 g (1 Mol) Cyclododecanon, gelöst in 200 ml Toluol, und 10 g Tetra-butyl-ammonium-bromid vorgelegt. Es wird innerhalb einer Stunde unter Rühren bei einer Temperatur von 70 bis 80 °C des Reaktionsgemisches 1 Mol Allylchlorid zugetropft. Danach wird noch weitere 5 Stunden bei 80 °C gerührt, schließlich dem Reaktionsgemisch 200 ml Wasser zugesetzt und die Phasen getrennt. Die organische Phase wird nun mit weiteren 100 ml Wasser neutralgewaschen und anschließend destilliert. Nach Abziehen der Niedrigsieder destilliert im Vorlauf zunächst nicht umgesetztes Cyclododecanon. Schließlich wird bei 0,01 Torr und 82 bis 84 °C in einer Ausbeute von 149,8 g, entsprechend 67,5 % der Theorie, das gewünschte 2-(2-Propenyl)-cyclododecanon als farblose Flüssigkeit erhalten.

b) In einem Dreihalskolben mit Rührer und Vigreux-Kolonne wird 0,1 Mol Aluminiumisopropylat in 500 ml Isopropanol vorgelegt. Es wird unter Rückfluß gekocht. In das siedende Reaktionsgemisch wird innerhalb von 12 Stunden eine 25 Gew.%-ige Lösung von 2-(2-Propenyl)-cyclododecanon in Isopropanol zugetropft. Am Kopf der Kolonne wird das entstandene Aceton, das zugleich als Indikator für den Fortgang der Reaktion dient, gesammelt. Nach noch ca. einstündigem Rückflußkochen ist die Reaktion beendet. Nach Abziehen des Isopropanols wird das Reaktionsgemisch mit 6 n Salzsäure hydrolysiert. Danach wird mit 200 ml Toluol extrahiert und die organische Phase destillativ aufgearbeitet. Schließlich wird in einer Ausbeute von 95 % der Theorie 2-(2-Propenyl)-cyclododecanol als farblose Flüssigkeit im Siedeintervall von 172 bis 175 °C bei 12 Torr erhalten.

c) 1 Mol 2-(2-Propenyl)-cyclododecanol wird zusammen mit 20 g p-Toluolsulfonsäure in 1 000 ml Toluol gelöst und 15 Stunden unter Rückfluß gekocht. Danach wird mit 10 %-iger Sodalösung neutralisiert, die Phasen getrennt und die organische Phase destillativ aufgearbeitet. Nach Abziehen des Toluols wird in einer Ausbeute von 98 % der Theorie im Siedeintervall von 85 bis 88 °C bei 0,05 Torr 14-Methyl-13-oxabicyclo [10.3.0] pentadecan erhalten.

Farbloses Öl ; Geruch : warmer, vetiverartiger Sandelholzton.

## Beispiel 2

Es wird die Arbeitsweise gemäß Beispiel 1 wiederholt, mit der Abänderung, daß anstatt mit Isopropanol/Aluminiumisopropylat (gemäß b) mit Natriumboranat reduziert wird.

1 Mol 2-(2-Propenyl)-cyclododecanon wird unter Rühren bei Raumtemperatur innerhalb von 1 Stunde zu einer Lösung von 15,2 g (0,4 Mol) Natriumborhydrid, gelöst in 1 200 ml Isopropanol, zugetropft. Danach wird noch unter Rühren 2 Stunden unter Rückfluß gekocht. Nach Abziehen des überschüssigen Isopropanols wird das Reaktionsgemisch mit 2 n Salzsäure bis zur Beendigung der Wasserstoffentwicklung versetzt. Danach wird mit Ether extrahiert, die organische Phase mit Natriumsulfat getrocknet und nach Abziehen des Ethers destilliert.

In einer Ausbeute von 95 % der Theorie wird das gewünschte 2-(2-Propenyl)-cyclododecanol als farblose, hochviskose Flüssigkeit, die bei Raumtemperatur langsam zu einer wachsartigen Masse erstarrt, erhalten.

## Beispiele 3 bis 6

Analog der Arbeitsweise gemäß Beispiel 1 werden die folgenden erfindungsgemäßen Verbindungen erhalten :

14.14-Dimethyl-13-oxabicyclo [10.3.0] pentadecan,
farbloses Öl ; Kp bei 12 Torr 165 bis 168 °C.
Geruch : milder Sandelholzton mit Vetivernote.
14-Ethyl-13-oxabicyclo [10.3.0] pentadecan,
farbloses Öl ; Kp bei 0,05 Torr 88 bis 90 °C.
Geruch : warme Sandelholz-Ambra-Note.

14.15-Dimethyl-13-oxabicyclo [10.3.0] pentadecan, farbloses Öl ; Kp bei 12 Torr 166 bis 167 °C.
Geruch : starke, rauchig-holzige Moschusnote.
14.14-Dimethyl-13-oxabicyclo [10.4.0] hexadecan, farblose, wachsartig erstarrende Masse ; Kp bei 0,03 Torr 88 bis 91 °C.
Geruch : animalisch-holzige Note.

**Patentansprüche**

1. Verbindungen der allgemeinen Formel I

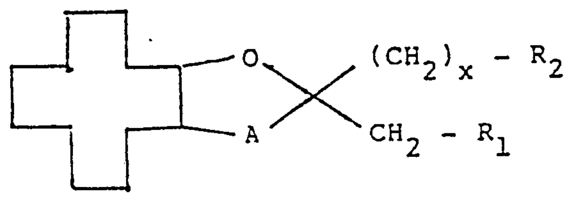

(I)

in der

$R_1$ und $R_2$ Wasserstoff oder ggf. verzweigte Alkylgruppen mit 1 bis 6 Kohlenstoffatomen oder ggf. verzweigte Alkenylgruppen mit 3 bis 6 Kohlenstoffatomen bedeuten,

A für einen zweiwertigen Kohlenwasserstoffrest der allgemeinen Formel II

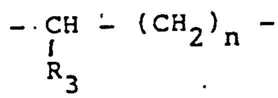

(II)

steht, in der

$R_3$ Wasserstoff oder Methyl bedeutet und

n und x den jeweils gleichen numerischen Wert von 0 oder 1 haben.

2. Verbindungen nach Anspruch 1, dadurch gekennzeichnet, daß $R_1$ und $R_2$ jeweils für Wasserstoff oder Methyl stehen.

3. Verfahren zur Herstellung von Verbindungen nach Anspruch 1, dadurch gekennzeichnet, daß

a) mit der Maßgabe, daß n und x identisch 0 sind, Verbindungen der allgemeinen Formel III

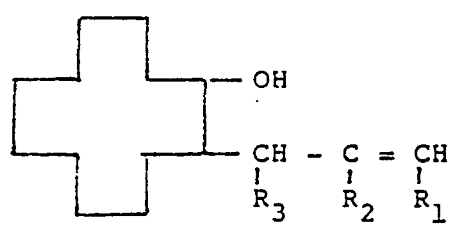

(III)

b) mit der Maßgabe, daß n und x identisch 1 sind, Verbindungen der allgemeinen Formel IIIa

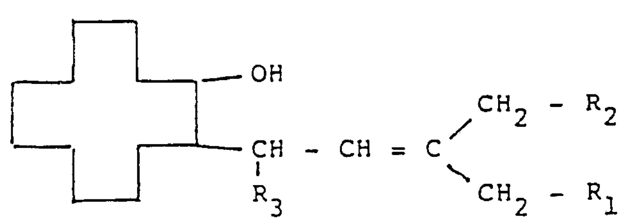

(IIIa)

in denen $R_1$, $R_2$ und $R_3$ jeweils die obengenannte Bedeutung haben, bei Temperaturen von 20 bis 150 °C mit einer Säure behandelt werden.

4. Verfahren zur Herstellung von Verbindungen nach Anspruch 1, dadurch gekennzeichnet, daß

a1) mit der Maßgabe, daß x und n identisch 0 sind, Cyclododecanon mit einem allylischen Halogenid der allgemeinen Formel IV

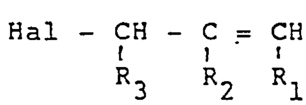

(IV)

in der Hal für ein Chlor-, Brom- oder Jodatom steht

a2) mit der Maßgabe, daß x und n identisch 1 sind, Cyclododecanon mit einem allylischen Halogenid der allgemeinen Formel V

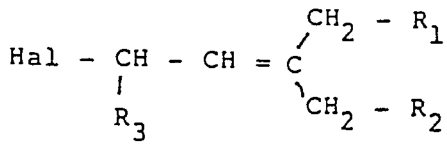

(V)

in der Hal für ein Chlor-, Brom- oder Jodatom steht, in einem organisch/alkalischen 2-Phasen-System in Gegenwart eines Phasentransferkatalysators umgesetzt wird,

b) das Reaktionsprodukt gemäß a1) bzw. a2) mit Isopropanol in Gegenwart von Aluminiumisopropylat zum entsprechenden Alkohol reduziert wird und

c) das Reaktionsprodukt gemäß b) bei Temperaturen von 20 bis 150 °C einer Säurebehandlung unterworfen wird.

5. Verwendung der Verbindungen nach Anspruch 1 als Riechstoffe.

## Claims

1. Compounds of the general formula I

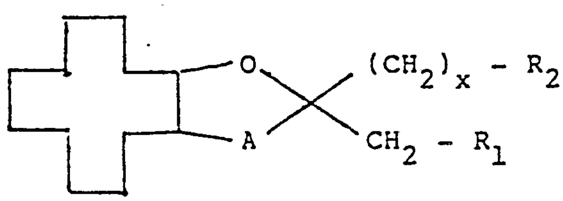

(I)

in which

$R_1$ and $R_2$ denote hydrogen or optionally branched alkyl groups having 1 to 6 carbon atoms or optionally branched alkenyl groups having 3 to 6 carbon atoms,

A represents a divalent hydrocarbon radical of the general formula II

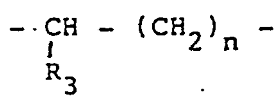

(II)

in which

$R_3$ denotes hydrogen or methyl and

n and x each have the same numerical value of 0 or 1.

2. Compounds according to Claim 1, characterized in that $R_1$ and $R_2$ each represent hydrogen or methyl.

3. Process for the preparation of compounds according to Claim 1, characterized in that

a) with a proviso that n and x are identical with 0, compounds of the general formula III

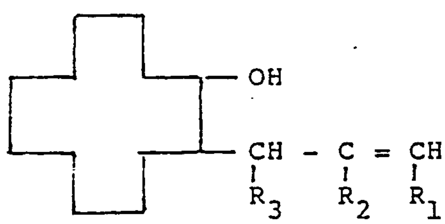

(III)

b) with the proviso that n and x are identical with 1, compounds of the general formula IIIa

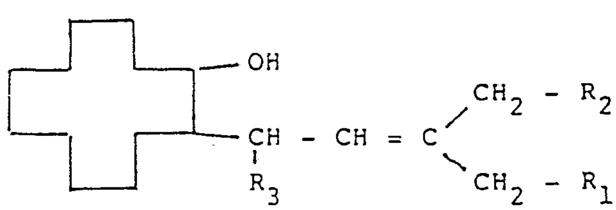

(IIIa)

in which $R_1$, $R_2$ and $R_3$ each have the abovementioned meaning, are treated with an acid at temperatures of from 20 to 150 °C.

4. Process for the preparation of compounds according to Claim 1, characterized in that

a1) with the proviso that x and n are identical with 0, cyclododecanone is reacted with an allylic halide of the general formula IV

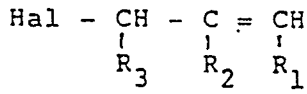

(IV)

in which Hal represents a chlorine, bromine or iodine atom,

a2) with the proviso that x and n are identical with 1, cyclododecanone is reacted with an allylic halide of the general formula V

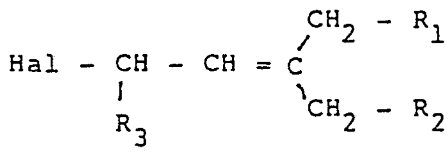

(V)

in which Hal represents a chlorine, bromine or iodine atom, in an organic/alkaline 2-phase system in the presence of a phase-transfer catalyst,

b) the reaction product from a1) or a2) is reduced using isopropanol in the presence of aluminium isopropylate to form the corresponding alcohol, and

c) the reaction product from b) is subjected to acid-treatment at temperatures of from 20 to 150 °C.

5. Use of the compounds according to Claim 1 as fragrances.

**Revendications**

1. Composés répondant à la formule générale I :

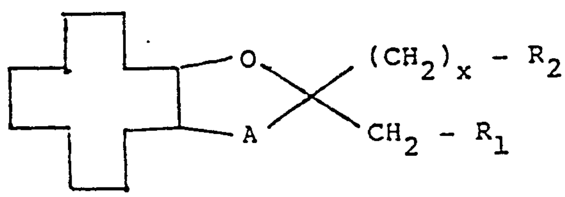

(I)

dans laquelle :

$R_1$ et $R_2$ représentent chacun l'hydrogène, un radical alkyle, éventuellement ramifié, qui contient de 1 à 6 atomes de carbone, ou un radical alcényle, éventuellement ramifié, contenant de 3 à 6 atomes de carbone,

A représente un radical hydrocarboné bivalent qui répond à la formule générale II :

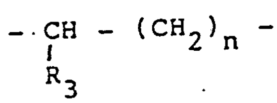

(II)

$R_3$ représente l'hydrogène ou un radical méthyle et

n et x sont égaux l'un à l'autre et représente chacun 0 ou 1.

2. Composés selon la revendication 1, caractérisés en ce que $R_1$ et $R_2$ représentent chacun l'hydrogène ou un méthyle.

3. Procédé de préparation de composés selon la revendication 1, procédé caractérisé en ce que :

a) dans le cas où n et x sont égaux l'un et l'autre à 0 on traite des composés répondant à la formule générale III :

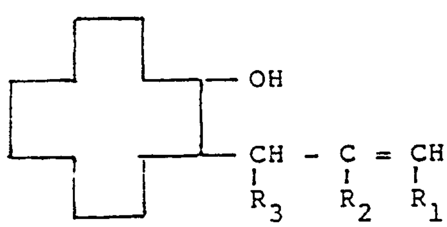

(III)

b) dans le cas où n et x sont égaux chacun à 1 on traite des composés de formule générale IIIa :

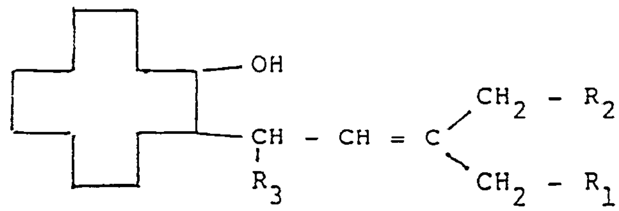

(IIIa)

formules dans lesquelles $R_1$, $R_2$ et $R_3$ ont chacun les significations qui leur ont été données ci-dessus, par un acide, à des températures de 20 à 150 °C.

4. Procédé de préparation de composés selon la revendication 1, procédé caractérisé en ce que :

a1) dans le cas où x et n sont égaux l'un et l'autre à 0 on fait réagir la cyclododécanone avec un halogénure d'allyle répondant à la formule générale IV :

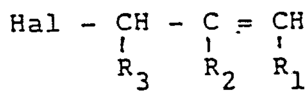

(IV)

dans laquelle Hal représente un atome de chlore, de brome ou d'iode,

a2) dans le cas où x et n sont égaux l'un et l'autre à 1 on fait réagir la cyclododécanone avec un halogénure d'allyle répondant à la formule générale V :

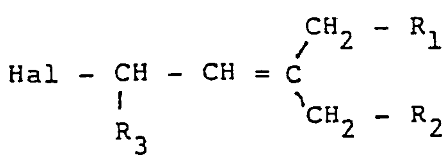

(V)

dans laquelle Hal représente un atome de chlore, de brome ou d'iode, dans un système à deux phases phase organique/phase alcaline, en présence d'un catalyseur de transfert de phase,

b) on réduit le produit réactionnel selon a1) ou a2) avec l'isopropanol en présence d'isopropylate d'aluminium de manière à le convertir en l'alcool correspondant, et

c) on soumet le produit réactionnel selon b) à un traitement acide à des températures de 20 à 150 °C.

5. Application des composés selon la revendication 1 comme parfums.